# DEMANDE DE BREVET EUROPEEN

(11) **EP 2 692 259 A1**
(43) Date de publication de la demande: **05.02.2014**
(21) Numéro de dépôt: 13003699.9
(22) Date de dépôt: 23.07.2013
(51) Int. Cl.: A42B 3/12, A42C 2/00

(54) **Casque de protection pour activité sportive**

(30) Priorité: 01.08.2012 FR 1202159
(71) Demandeur: SALOMON S.A.S., 74370 Metz-Tessy (FR)
(72) Inventeur: Donnadieu, Thierry, 74330 Poisy (FR); Guyoton, Alexis, 38240 Revel (FR)

(57) **Abrégé**

L'invention concerne un casque d'amortissement (1, 101, 201) pour activité sportive comprenant au moins une couche d'amortissement (3) couvrant une partie du crâne (71) d'un utilisateur et une coiffe (4, 104, 204) destinée à être interposée, au moins partiellement, entre ladite couche de protection (3) et le crâne (71), la coiffe (4,104, 204) comportant au moins une poche (40, 1040, 2040).

Le casque est caractérisé en ce que le volume interne de la poche définit, dans une configuration initiale de personnalisation, au moins localement, une épaisseur initiale de coiffe (Ei) et en ce que le casque (1) comprend un moyen de réglage (5, 105, 205) du volume interne de la poche permettant, suite à une compression de la poche, l'évacuation d'une partie du fluide contenu dans la poche, cette diminution du volume interne de la poche assurant une réduction localisée de l'épaisseur initiale de coiffe afin de conformer la coiffe à la morphologie du crâne.

## Description

L'invention concerne un casque de protection pour activité sportive. Notamment mais, non exclusivement, l'invention concerne un casque pour la pratique de sports, notamment alpins, par exemple du ski, du snowboard, mais aussi du cyclisme, de l'alpinisme, de l'escalade, du hockey sur glace, du patin à glace, du football, du patin à roulettes.

De façon connue, un casque comprend des moyens de protection de la tête qui protège le crâne des chocs qu'il pourrait subir lorsque l'utilisateur fait une chute ou une collision ou lorsqu'un objet est projeté dans sa direction. Les moyens de protection peuvent être constitués d'une coque et d'une calotte amortissante. La coque peut être rigide. Par exemple, elle est réalisée par moulage/injection d'une matière thermoplastique telle que l'ABS (Acrylonitrile Butadiène Styrène). Alternativement, la coque peut n'être qu'une peau extérieure n'ayant en soi pas de rigidité, mais en l'acquérant dès lors que cette peau est liée à la calotte amortissante. Par exemple la peau peut être réalisée par mise en forme d'une feuille de PVC (PolyVinyl Chloride) ou de PC (PolyCarbonate). De manière courante, la calotte amortissante est réalisée dans un matériau expansé tel que l'EPS (Expansed PolyStyrène) ou encore l'EPP (Expansed PolyPropylène). Afin de garantir un bon confort à l'utilisateur, on équipe le casque de moyens de confort. Ceux-ci peuvent prendre la forme d'une coiffe interne faite, par exemple, de mousse recouverte de tissu.

Pour permettre une adaptation du volume interne du casque à la tête de l'utilisateur, de nombreux dispositifs sont connus de l'art antérieur.

Le document US 6,647,556 propose d'adapter le volume par glissement les unes par rapport aux autres des différentes portions constituant le casque. Un tel dispositif est difficile à régler et rend le casque relativement lourd à porter pour l'utilisateur.

Une autre solution est donnée par le document FR 2 888 729 dans lequel on propose de modifier le volume intérieur du casque en gonflant une poche d'une coiffe interne à l'aide d'une pompe manuelle. La poche est initialement dégonflée. Le volume interne de la poche est sensiblement nul. L'épaisseur de la coiffe est fine et correspondant à celle des parois de la poche. La circonférence de la coiffe est supérieure à la circonférence du crâne de l'utilisateur. En conséquence, l'utilisateur peut enfiler facilement le casque du fait du jeu entre la coiffe et le crâne. Pour adapter la coiffe à la morphologie du porteur, celui-ci va gonfler la poche, à l'aide de la pompe manuelle, jusqu'à ce que la coiffe soit calée. Avantageusement, la coiffe intègre une soupape d'échappement d'air, actionnable manuellement, permettant l'évacuation de l'air de la poche pour que le casque ne soit plus adapté à la morphologie du crâne de l'utilisateur. Un tel dispositif n'est pas optimal à l'usage étant donné qu'il faut adapter le volume intérieur du casque, par une action de gonflage manuelle, quasiment à chaque fois que l'on porte ce casque. Le gonflage peut prendre du temps selon la morphologie de crâne. D'autre part, si la pression à l'intérieur de la poche est trop importante, il peut en découler une gêne pour l'utilisateur. En effet, et cela est vrai pour tous les dispositifs d'adaptation de volume, il faut à tout prix éviter que le casque exerce localement une pression sur la tête de l'utilisateur provoquant un inconfort. L'ajustement de la pression exercée sur le crâne n'est pas évident avec ce système car il est réalisé par une insufflation définie, propre à la pompe. Ce principe actif d'ajustement de volume ne permet pas un réglage rapide et automatique du casque à la morphologie du crâne de l'utilisateur.

Compte-tenu des inconvénients de l'art antérieur, il existe un besoin de proposer un casque de protection, notamment pour la pratique sportive, qui comporte des moyens de personnalisation de son volume intérieur, qui soient rapides, faciles d'utilisation, peu volumineux, peu coûteux et qui s'adaptent à tous types de morphologie du crâne. Avantageusement, ces moyens de personnalisation sont automatiques.

L'objectif de l'invention est atteint par la fourniture d'un casque pour activité sportive comprenant au moins une couche d'amortissement couvrant une partie du crâne d'un utilisateur et une coiffe destinée à être interposée, au moins partiellement, entre ladite couche d'amortissement et le crâne, la coiffe comportant au moins une poche.

Le casque est caractérisé en ce que le volume interne de la poche définit, dans une configuration initiale de personnalisation, au moins localement, une épaisseur initiale de coiffe et en ce que le casque comprend un moyen de réglage du volume interne de la poche permettant, suite à une compression de la poche, l'évacuation d'une partie du fluide contenu dans la poche, cette diminution du volume interne de la poche assurant une réduction localisée de l'épaisseur initiale de coiffe afin de conformer la coiffe à la morphologie du crâne.

Ainsi, la poche de la coiffe du casque est à l'état gonflé avant la personnalisation. Puis, pour adapter le casque à la morphologie de la tête, il suffit seulement d'enfoncer le casque sur la tête jusqu'à une position confortable ce qui crée une surpression qui a pour conséquence que l'air en surpression s'échappe par le moyen de réglage. Celui-ci empêche ensuite à la poche de se regonfler et l'intérieur du casque garde la forme ainsi « imprimée » de la tête. La coiffe est conformée à la morphologie du crâne.

Le casque selon l'invention peut comporter une ou plusieurs des caractéristiques suivantes prises seules ou en combinaison.
- Le moyen de réglage comprend un dispositif permettant de maintenir une dépression du volume interne de la poche par rapport à la pression du volume interne de la poche lorsqu'elle est dans une configuration initiale de personnalisation.
- Le volume interne de la poche est à pression ambiante, lorsque la poche est dans sa configuration initiale.
- Le moyen de réglage comprend un dispositif permettant l'évacuation automatique d'une partie du fluide contenu dans la poche suite à une compression de la poche.
- Le moyen de réglage comprend une valve anti-retour. Selon un mode de réalisation, le moyen de réglage comprend un moyen d'inhibition de la valve anti-retour permettant une fonction évent pour le regonflage de la poche. Selon une variante, seul le moyen d'inhibition permet le regonflage de la poche en connectant le volume interne de la poche à l'air ambiant.
- La coiffe comprend un moyen de regonflage automatique de la poche. Préférentiellement, ce moyen de regonflage automatique est une mousse comprimable. Selon un mode de réalisation, la mousse comprimable est emprisonnée soit par deux panneaux soudés l'un à l'autre, soit par un panneau replié dont les bords sont soudés les uns aux autres, soit par un manchon soudé à ses extrémités. Préférentiellement, la densité de la mousse comprimable est comprise entre 20 kg/m3 et 50 kg/m3. Préférentiellement, l'épaisseur de la mousse comprimable est comprise entre 5 mm et 12 mm.
- La coiffe couvre une bande sensiblement circonférentielle à l'intérieur du casque correspondant à un tour de tête total ou partiel d'un utilisateur.
- Le casque comporte une chambre de gonflage disposée au niveau de la partie du casque destinée à être en contact avec la partie sous-occipital du crâne de l'utilisateur, et en ce qu'un élément du moyen de réglage de la poche est relié à cette chambre de gonflage pour gonfler celle-ci, au moins partiellement, en cas de réduction volume du fluide contenu dans la poche lors de l'adaptation au moins partielle du casque à la morphologie du crâne de l'utilisateur.

L'invention concerne en outre un procédé de personnalisation d'un casque de protection à la morphologie du crâne d'un utilisateur comprenant les étapes suivantes :
- Initialiser la poche dans sa configuration initiale, dans cette configuration la circonférence interne de la coiffe est inférieure à la circonférence du crâne au niveau des zones de contact,
- Mise en place du casque sur la tête de l'utilisateur entrainant l'évacuation automatique, ou par une action manuelle, d'une partie du fluide contenu dans la poche suite à la compression de la poche afin de conformer la coiffe à la morphologie du crâne.

D'autres avantages et caractéristiques apparaîtront à la lecture de la description de l'invention, ainsi que des figures suivantes parmi lesquelles :
- la figure 1 est un schéma en coupe d'un casque selon l'invention,
- la figure 2 est un schéma en coupe d'un casque selon l'invention placé sur la tête d'un utilisateur lors de l'adaptation du casque à la morphologie de la tête de l'utilisateur,
- la figure 3 est un schéma en coupe d'un casque selon l'invention lors de la réinitialisation de la coiffe du casque,
- la figure 4 est un schéma en coupe d'un deuxième mode de réalisation d'un casque selon l'invention,
- la figure 5 est un schéma en coupe d'un troisième mode de réalisation d'un casque selon l'invention,
- la figure 6 un organigramme schématique pour illustrer les diverses étapes de réalisation du coussinet pour un casque selon l'invention.

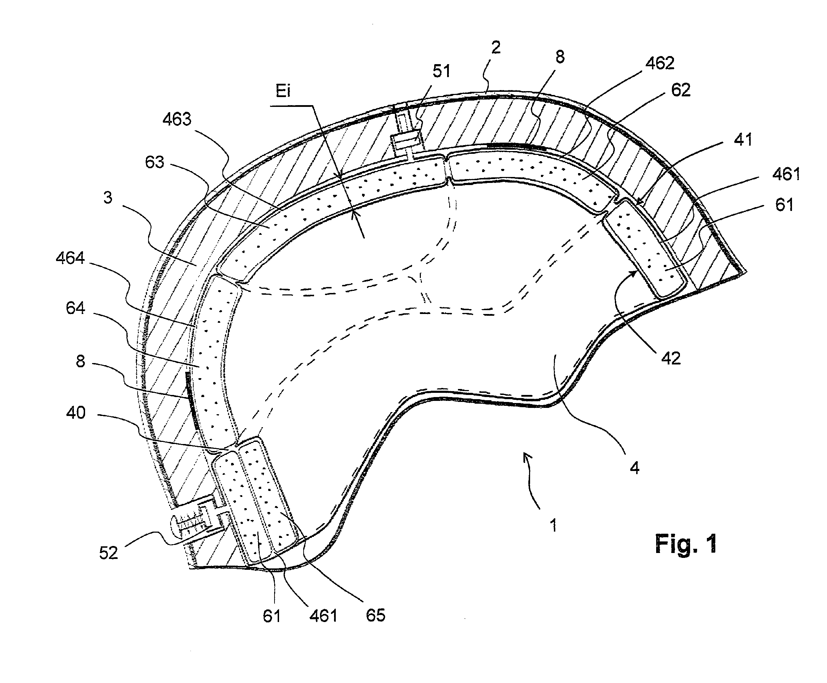

Sur la figure 1 est représenté en coupe un casque de protection 1 pour activité sportive, notamment des sports alpins et plus particulièrement des sports de glisse comme par exemple du ski, du snowboard etc.

Ce casque 1 comprend plusieurs couches de protection. Dans cet exemple, il comprend une coque 2 et au moins une couche d'amortissement 3, appelée également calotte, revêtant l'intérieur de la coque 2. Cette calotte 3 est destinée à couvrir une partie du crâne 71 de l'utilisateur.

La coque 2 est par exemple réalisée par moulage/injection d'une matière thermoplastique telle que l'ABS (Acrylonitrile Butadiène Styrène) ou le PC (PolyCarbonate).

La calotte 3 est par exemple réalisée dans un matériau expansé tel que l'EPS (Expansed PolyStyrène) ou encore l'EPP (Expansed PolyPropylène). Il peut s'agir d'une seule couche ou de plusieurs couches identiques ou différentes assemblées.

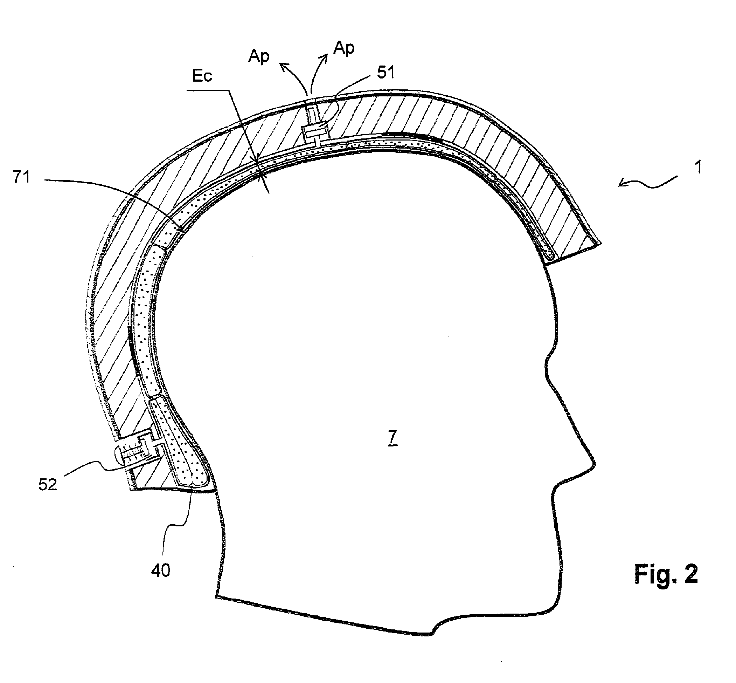

De plus, le casque 1 comprend une coiffe 4 destinée à être interposée entre la calotte 3 et la tête 7 d'un utilisateur de casque (voir figure 2). La coiffe 4 comporte une poche 40 apte à recevoir un fluide, en l'occurrence de l'air dans cet exemple.

Il convient de distinguer la calotte 3 de la coiffe 4. La calotte 3 assure l'amortissement du choc. Grâce à cette couche, l'impact est amorti. La couche d'amortissement joue ainsi un rôle protecteur direct de la tête. Selon notre invention, la coiffe 4 est un élément de confort qui agit peu sur la protection de la tête. Cette couche supplémentaire améliore le portage du casque en permettant de mieux caler les couches de protection autour du crâne de l'utilisateur.

Le casque 1 comprend également un moyen de réglage 5 du volume interne de la poche. Ce moyen de réglage 5 est relié à la poche 40. Il comporte une valve anti-retour 51 permettant l'évacuation d'une partie de l'air contenu dans la poche 40 suite à une compression de la poche. Cette valve laisse l'air à l'intérieur de la poche 40 s'échapper par exemple vers l'extérieur (voir flèches Ap figure 2), mais elle empêche l'air extérieur de rentrer dans la poche. Cette valve permet une adaptation au moins partielle du casque à la morphologie de la tête de l'utilisateur comme cela va être expliqué par la suite.

Pour personnaliser le casque à la morphologie de son porteur, celui-ci va procéder aux étapes suivantes :

Lors d'une première étape, l'utilisateur initialise la poche 40 de manière qu'un certain volume d'air, non nul, remplisse la poche. Ce volume permet de créer, au moins localement, une épaisseur de coiffe Ei. Cette épaisseur Ei peut être différente à l'avant, au sommet ou à l'arrière de la coiffe. Dans cette configuration, l'enveloppe interne de la coiffe 4 est inférieure à l'enveloppe externe du crâne 71. De même, la circonférence interne de la coiffe est inférieure à la circonférence du crâne au niveau des zones de concordance.

Lors d'une deuxième étape, l'utilisateur insère sa tête 7 dans le casque jusqu'à ce la coiffe se conforme à la morphologie du crâne de l'utilisateur. Cette action crée une surpression dans la poche 40. L'air de la poche 40 est alors évacué par la valve anti-retour 51, c'est-à-dire, un élément du moyen de réglage 5. Cette valve anti-retour assure la circulation de l'air uniquement dans un sens, à savoir, le dégonflage de la poche. La valve anti-retour empêche la poche 40 de se regonfler. Elle permet donc réduire le volume interne de la poche suite à une compression de la poche. Cette diminution de volume provoque une réduction au moins localisée de l'épaisseur de coiffe. L'intérieur du casque garde alors la forme ainsi « imprimée » de la tête 7.

Ces opérations présentent l'avantage d'être simples et très rapides à faire. L'ajustement se fait en même temps que l'utilisateur met son casque. Il s'agit d'un principe passif dans le sens où il n'y a pas besoin d'une action spécifique supplémentaire de personnalisation après la mise en place du casque sur la tête, contrairement aux dispositifs de l'art antérieur où un gonflage manuel de la poche est nécessaire. La personnalisation se fait simultanément avec la mise en place du casque sur la tête, ce qui permet un gain de temps. De plus, cette personnalisation permet une conformation précise de la coiffe à la morphologie du crâne de l'utilisateur. La deuxième étape permet ainsi de créer une empreinte juste du crâne sur la coiffe ce qui apporte une amélioration du confort de portage.

Concernant la valve anti-retour 51, celle-ci peut être actionnée manuellement par un bouton, en dévissant un bouchon ou par tout autre moyen. Dans une variante, la valve 51 n'est pas une valve anti-retour. Dans ce cas, le blocage de la circulation d'air est obtenu par le déclencheur de la valve. Par exemple, la valve peut être une simple vanne. Avantageusement, la valve anti-retour est à déclenchement automatique, en étant, par exemple, énergisée. Cette valve forme ainsi un dispositif assurant l'évacuation automatique de l'air dès lors que la pression interne dépasse un seuil, suite à la compression de la poche. Cet aspect automatique facilite davantage la personnalisation puisqu'il n'y a plus besoin d'une action particulière de l'utilisateur pour déclencher la conformation de la coiffe. Le porteur doit seulement mettre le casque après avoir initialiser la poche, conformément à la première étape. Dans les modes de réalisation décrits ci-après, la valve anti-retour 51 est automatique.

Pour initialiser la poche plusieurs solutions sont envisageables.

Une première solution consiste à lier la poche à une pompe. Ainsi, l'utilisateur va gonfler le volume interne de la poche avec la pompe, pour obtenir une pression de gonflage initial dans la poche. Lorsque l'utilisateur va enfiler le casque, il va appuyer sur la poche ce qui engendre une surpression de l'air contenu dans la poche. L'air est alors évacué par la valve anti-retour tarée sur une pression prédéterminée, supérieure ou égale à la pression de gonflage initial. Le volume interne de la poche diminue ce qui permet l'adaptation morphologique souhaitée. L'air restant dans le volume interne réduit de la poche est toujours sous pression.

Une deuxième solution, décrite dans les figures 1 à 6, consiste à intégrer, dans le casque, des moyens de mise en forme permettant de maintenir un volume interne initial déterminé lorsque le volume interne de la poche est à pression ambiante. En conséquence, simplement en connectant la poche à l'air ambiant, on assure un certain volume interne de la poche se traduisant par une épaisseur initiale de coiffe Ei, non nulle, au moins localement. Cette configuration correspond à la configuration initiale de personnalisation.

Lors de la mise en place du casque sur la tête, le crâne va appuyer sur la poche ce qui engendre une surpression de l'air contenu dans la poche. L'air est alors évacué par la valve anti-retour relié à la poche et le volume interne de la poche diminue ce qui permet l'adaptation morphologique souhaitée, au moins partiellement. A la différence de la première solution, l'air restant dans la poche au volume interne réduit est en dépression. Pour cela, il faut donc que la valve anti-retour 51, un élément du moyen de réglage 5, permette de maintenir une dépression du volume interne suite à une compression de la poche. C'est cette dépression qui assure la réduction localisée de l'épaisseur de coiffe par la réduction du volume interne.

Pour initialiser la poche, il faut simplement connecter la poche à l'air ambiant. Dès lors, les moyens de mise en forme agissent sur le volume interne de la poche pour qu'il atteigne le volume interne initial déterminé. Pour cela, le moyen de réglage 5 comprend également un moyen d'inhibition 52 de la valve anti-retour 51 permettant une fonction évent pour le regonflage de la poche. Les moyens de mise en forme décrits précédemment correspondent à des moyens de regonflage automatique de la poche car il permettre de redonner à la poche son volume interne initial correspondant à la configuration initiale de personnalisation.

Cette deuxième solution est avantageuse car elle peut être compacte et facilement intégrable dans le casque car il n'y a pas besoin de pompe, le moyen d'inhibition pouvant être une simple valve anti-retour actionnable manuellement. De plus, elle est facile à utiliser car l'action de l'utilisateur est simple, appuyer sur un bouton versus gonfler la poche par des pressions successives.

D'autres solutions sont possibles. On peut, par exemple, prévoir une coiffe à personnalisation unique. Dans ce cas, le moyen de réglage ne permet pas l'initialisation de la coiffe. La coiffe dispose d'un volume interne initial. La mise en place du casque chasse l'air en surpression pour que la coiffe se conforme à la morphologie de la tête de l'utilisateur. Le casque est alors réglé sans possibilité de le réinitialiser.

Par la suite, nous allons détailler des modes de réalisation correspondant à la deuxième solution décrite précédemment et aux figures 1 à 6.

Selon le premier mode de réalisation, représenté par les figures 1 à 3, la coiffe 4 comprend deux panneaux 41 et 42, l'un 41 plus proche de la calotte 3, l'autre 42 destiné être plus proche de la tête 7. Ces deux panneaux 41 et 42 peuvent être réalisés en matière plastique ou en tissu incorporant un film étanche et un film thermocollant afin d'améliorer le contact avec le crâne de l'utilisateur et donc le confort de portage. Ces deux panneaux 41 et 42 sont soudés l'un à l'autre pour former la poche étanche 40 emprisonnant entre eux au moins une mousse comprimable 61, 62, 63, 64. Cette mousse comprimable constitue le moyen de mise en forme ou, autrement dit, le moyen de regonflage automatique de la poche.

D'autres solutions peuvent être envisagées pour réaliser la poche 40. Ce peut être un seul panneau replié dont les bords sont soudés les uns aux autres ou un manchon soudé à ses extrémités. Alternativement, ce peut être un panneau directement fixé sur la paroi interne étanchéifiée de la calotte 3.

Dans l'exemple illustré à travers les figures 1 à 3, la coiffe 4 couvre toute la partie supérieure du crâne 71. La poche 40 contient quatre mousses distinctes 61, 62, 63, 64. Pour maintenir ces mousses en place dans la coiffe 4, celle-ci peut comprendre des chambres 461, 462, 463, 464, communicant entre elles par des ouvertures, délimités par des lignes de soudure interrompues entre les deux parois de la poche, c'est-à-dire, les panneaux 41, 42. En variante, la coiffe 4 peut comprendre plusieurs poches 40 distinctes reliées par des conduits. Il importe alors que les éléments 51, 52 du moyen de réglage 5 soit connectés à ce réseau de chambres ou poches.

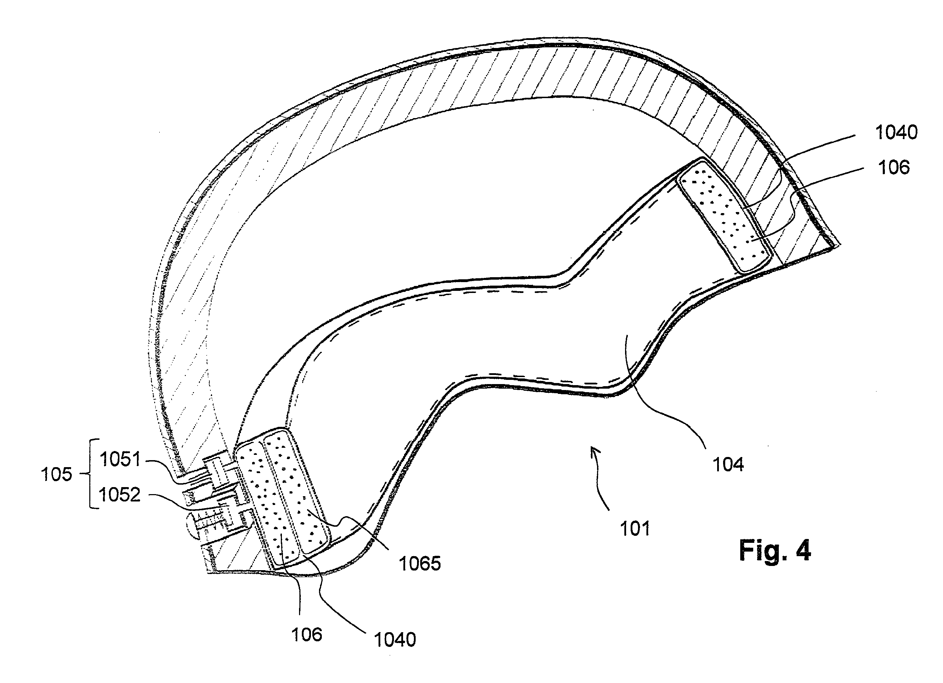

La figure 4 illustre un autre mode de réalisation dans lequel une coiffe 104 couvre une bande sensiblement circonférentielle à l'intérieur du casque 101 correspondant à un tour de tête total d'un utilisateur. Cette configuration permet une meilleure ventilation du crâne. Par ailleurs, on s'est aperçu que cette zone interface est suffisante pour assurer un maintien confortable et suffisant du casque. Dans ce cas, il n'y a qu'une seule poche 1040 dans laquelle se loge une mousse 106.

Pour que la personnalisation soit efficace, le choix de la mousse comprimable est déterminant. De bons résultats sont obtenus avec une densité de mousse comprise entre 20 kg/m3 et 50 kg/m3 et/ou avec une épaisseur de mousse comprise entre 5 mm et 12 mm au moins sur le dessus du crâne et/ou sur la partie avant du crâne. Localement, il peut être avantageux d'avoir une épaisseur de mousse plus importante, comme par exemple, dans la zone arrière du crâne. Ces caractéristiques permettent d'avoir une amplitude d'adaptation suffisante sans que l'utilisateur ait l'impression de se tromper de taille lors du premier essai du casque 1. Avec cette configuration, la ou les mousses améliorent les sensations de confort de portage et d'adaptation morphologique de l'utilisateur lors de la personnalisation et en usage. La mousse est par exemple réalisée en polyuréthane.

On peut obtenir ainsi un effet mémoire de forme à l'intérieur du casque 1. En pressant le casque 1 sur la tête 7 (voir figure 2), on chasse l'air des alvéoles de la mousse 61, 62, 63, 64 vers l'extérieur, via la valve anti-retour 51. La poche 40 garde ainsi sa forme étant donné que la valve anti-retour 51 empêche l'air de rentrer dans poche 40. Le volume interne de la poche 40 est en dépression ce qui entraine la compression de la mousse 61, 62, 63, 64 par les parois de poche 40, c'est-à-dire, les panneaux 41, 42. La coiffe 4 reste aplatie.

Par sa faible densité et son élasticité, la mousse 61, 62, 63, 64 tend à écarter les deux panneaux 41 et 42, ce qui permet d'obtenir l'effet autogonflant de la poche 40 dès que celle-ci n'est plus en dépression.

Toutefois, étant donné que la poche, formée par les deux panneaux 41 et 42 est étanche, le gonflage ne peut se faire que si la valve anti-retour 51 est inhibée, c'est-à-dire qu'elle laisse entrer de l'air extérieur dans la poche. Sinon, l'épaisseur la poche 40 reste stable selon la pression interne de la poche. C'est pourquoi le moyen de réglage 5 comprend, comme évoqué précédemment, le moyen d'inhibition 52 de la valve anti-retour 51. Dans l'exemple illustré, la valve anti-retour 51 et le moyen d'inhibition 52 sont deux organes du moyen de réglage 5 distincts. Alternativement, Ces deux organes peuvent être combinés pour faire un seul dispositif. Dans ce cas, le moyen d'inhibition 52 agit au niveau de la valve anti-retour 51, en forçant par exemple, l'ouverture du clapet anti-retour.

Le moyen d'inhibition 52 permet une fonction évent pour le regonflage de la poche. Ainsi, cette inhibition supprime la dépression à l'intérieur de la poche 40. L'intérieur de la poche va revenir à pression ambiante. La mousse 61, 62, 63, 64, disposée à l'intérieur de la poche 40, tend alors à reprendre sa forme initiale car elle n'est plus comprimée par les parois 41, 42 de la poche, compression résultante de la dépression. Elle va donc repousser les parois de la poche. En conséquence, la mousse aide la poche à retrouver son volume initial. La coiffe et la poche reprennent leur configuration initiale de personnalisation, avec une épaisseur de coiffe associée Ei.

On peut envisager d'autres moyens de regonflage automatique ou de mise en forme de la poche que l'usage de mousse comprimable. Ce peut être des ressorts ou lames ressort. Ces moyens de regonflage automatique se caractérisent par un effet ressort permettant une augmentation du volume interne de la poche en vu de retrouver le volume interne initial de la poche.

Pour maintenir la coiffe 4, 104 contre la calotte 3 on prévoit une fixation autoagrippante 8. D'autres accroches sont possibles comme un bouton pression, des clips...

Avantageusement, pour améliorer la perception de l'utilisateur qu'il a correctement enfilé son casque et améliorer sa sensation de confort, une mousse supplémentaire 65 est superposée à la mousse inférieure 61 uniquement sur la partie postérieure de la coiffe, dans la zone occipitale. Cette superposition permet d'augmenter, localement, l'épaisseur de la coiffe. Cette surépaisseur améliore la proprioception de l'utilisateur. En effet, lorsque la partie occipitale du crâne 71 de porteur va franchir cette surépaisseur de mousse, celle-ci va naturellement se caler dans le creux arrière du crâne, sous l'os occipital. A ce moment, le casque va automatiquement s'ajuster dans une position confortable pour l'utilisateur. Celui-ci aura alors la sensation d'avoir passé un cran et de bien avoir mis son casque. Préférentiellement, cette mousse supplémentaire 65 découpée de manière ergonomique pour reproduire un contour proche de la morphologie du crâne dans cette zone occipitale. Cela permet de rendre plus uniforme la pression de maintien de la coiffe, et donc du casque, sur la tête, dans cette zone postérieure du crâne.

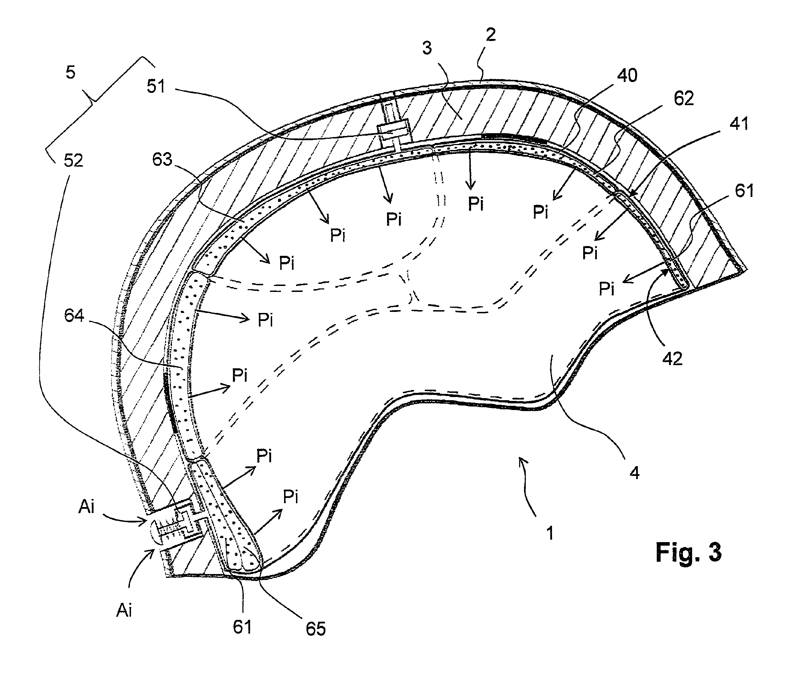

En résumé, la personnalisation selon l'invention appliquée au mode de réalisation des figures 1 à 3 est la suivante :
La première étape d'initialisation de la poche est illustrée à la figure 3. En actionnant le moyen d'inhibition 52, l'air ambiant pénètre dans la poche 40 (voir flèches Ai figure 3). Les mousses 61, 62, 63, 64, en reprenant leur forme originelle, exercent donc une pression sur les parois 41, 42 de la poche 40 (voir flèches Pi figure 3). La poche 40, autrement dit la coiffe 4, retrouve sa configuration initiale de personnalisation, comme illustré à la figure 1.
La deuxième étape, représentée à la figure 2, consiste à conformer la coiffe 4 à la morphologie du crâne 71 de l'utilisateur. Le casque 1 est enfoncé sur la tête 7 jusqu'à une position confortable. La poche 40 est comprimée, ce qui entraine la compression des mousses 61, 62, 63, 64. Le volume interne de la poche 40 est alors en surpression. L'air comprimé s'évacue alors par la valve anti-retour 51 (voir flèches Ap figure 2). En conséquence, le volume interne de la poche diminue et est en dépression, c'est-à-dire, à une pression inférieure à la pression ambiante. Cette dépression permet de maintenir la compression des mousses 61, 62, 63, 64. La coiffe 4 épouse alors la forme du crâne 71, dans ses zones de contact. Localement, l'épaisseur de coiffe Ec est alors inférieure à l'épaisseur initiale de coiffe Ei, dans sa configuration initiale de personnalisation. On distingue donc une configuration finale de personnalisation pour laquelle la coiffe est conformée à la morphologie du crâne, d'une configuration initiale de personnalisation pour laquelle la poche a repris un volume initial ou en est proche.

La figure 4 montre un autre mode de réalisation de la coiffe 104, sous forme d'une seule poche 1040 formant une bande périphérique logeant une mousse 106. Le principe de fonctionnement de la personnalisation reste identique. Une valve anti-retour 1051 et un moyen d'inhibition 1052 d'un moyen de réglage 105 sont directement connectés sur la poche 1040 de la coiffe 104. Une mousse supplémentaire 1065 est accolée à la mousse 106 dans la zone occipitale.

La personnalisation selon l'invention peut être répétée à souhait, par exemple pour prendre en compte la longueur de cheveux à des moments différents, un prêt entre utilisateurs ou en cas de location du casque. Il suffit alors d'actionner le regonflage automatique de la poche comme décrit ci-dessus.

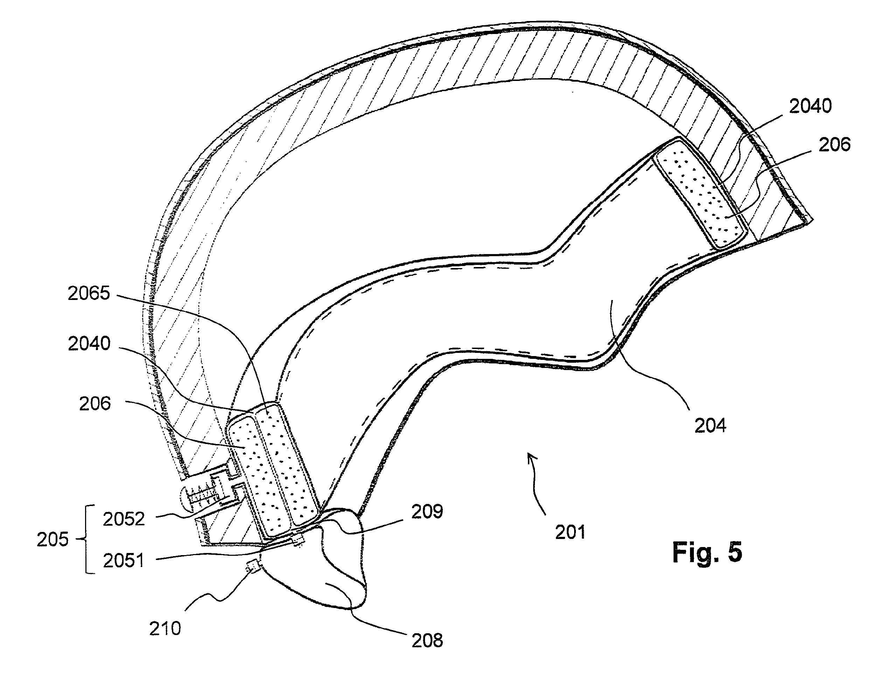

Sur la figure 5 est représenté un mode alternatif de réalisation d'un casque selon l'invention.

Ce casque 201 se distingue de celui de la figure 4 par le fait qu'il comporte, au niveau de la partie sous-occipitale du crâne 71 de l'utilisateur, une chambre de gonflage 208.

Comme précédemment, le casque 201 comprend une coiffe 204 intégrant une poche 2040 dans laquelle sont logés des mousses 206, 2065. Ce casque 201 comprend un moyen de réglage 205 du volume interne de la poche comportant une valve anti-retour 2051 et un moyen d'inhibition 2052 de la valve anti-retour.

Cependant, dans ce mode de réalisation, on prévoit que la valve anti-retour 2051 est reliée par un conduit 209 à la chambre de gonflage 208. Cette valve anti-retour 2051 permet de gonfler la chambre de gonflage 208, au moins partiellement, en cas de réduction d'état de gonflage de la poche 2040 lors de l'adaptation, au moins partielle, du casque à la morphologie de la tête 7 de l'utilisateur.

En conséquence, lorsqu'on chasse l'air de la poche 2040 en appuyant sur le casque 201 posé sur la tête 7, on gonfle automatiquement, au moins partiellement, la chambre de gonflage 208 pour la partie sous-occipitale du crâne 71. Cette chambre de gonflage 208 améliore sensiblement de confort d'utilisation en protégeant une partie de la nuque. La tête est alors mieux maintenue.

Le port du casque est ainsi assuré d'une part, par la poche 2040 dont le volume interne diminue pour accueillir la morphologie du crâne et d'autre part, par la chambre de gonflage 208 dont le volume interne augmente pour venir en contact avec la partie sous-occipitale du crâne. D'un coté, on obtient une empreinte négative, dans le sens où la forme du crâne s'inscrit dans la poche 2040 alors que de l'autre coté, on obtient une empreinte positive, dans le sens où le moyen de maintien 208 se déforme pour combler l'espace vide entre la coiffe et la tête.

Préférentiellement, on prévoit que le volume d'air de la poche 2040, à l'état entièrement gonflé, est supérieur au volume de la chambre de gonflage 208, à l'état gonflé, de préférence, compris entre 1,5 et 2 fois le volume de la chambre de gonflage 208, à l'état gonflé. Ceci prend en compte que l'on ne chasse pas l'air complètement de la poche 2040 lors de l'adaptation.

Pour éviter un éclatement de la chambre de gonflage 208, celle-ci est équipée d'une valve de surpression 210 qui permet d'évacuer le surplus d'air contenu dans la chambre de gonflage. La valve de surpression peut être une valve anti-retour automatique, une vanne manuelle ou un autre type de valve.

Au cas où la chambre de gonflage 208 n'est pas assez gonflée, on peut alors procéder à un gonflage complémentaire grâce à un gonfleur, non représenté, relié à la chambre de gonflage 208. Une telle construction est, par exemple, décrit dans le document FR 2 888 729.

D'autres variantes peuvent être envisagées pour ce mode de réalisation.

Par exemple, la poche 2040 peut également être reliée à l'air ambiant par une deuxième valve, en plus de la valve anti-retour 2051 relié à la chambre de gonflage 208. Cette deuxième valve permet notamment d'éviter un gonflement trop important de la poche 2040 si on bloque la valve anti-retour 2051.

Alternativement, la chambre de gonflage 208 intègre une mousse permettant la mise en forme de la chambre de gonflage pour s'approcher du crâne plus rapidement. Le fonctionnement du gonflage la chambre pourrait être analogue à celui de la poche 2040. La mousse ferait office de moyen de mise en forme tendant à agir sur les parois de la chambre de gonflage dès lors que la chambre revient vers une pression ambiante. Dans ce cas, la chambre de gonflage a été préalablement mise en dépression, par exemple, par une pression manuelle sur ses parois.

Une autre variante consiste à utiliser la chambre de gonflage 208 comme pompe pour ajuster le volume interne de la poche 2040. Ainsi, on ajoute une valve anti-retour reliant la chambre de gonflage à la poche. Cette valve permet d'évacuer le surplus d'air de la chambre de gonflage, suite à la compression de celle-ci, vers la poche. En conséquence, l'initialisation du casque peut être réalisée par la compression de la chambre de gonflage. L'air évacué de la chambre de gonflage vers la poche supprime, au moins partiellement, la dépression de la poche qui tend à reprendre sa forme initiale grâce à la mousse 206, 2065. Dès lors, la poche revient vers sa configuration initiale de personnalisation. De même, la chambre de gonflage tend à diminuer en volume ce qui va faciliter la mise en place du casque lors de sa prochaine personnalisation.

Les modes de réalisation précédents décrivent des poches gonflées à l'air. D'autres fluides peuvent aussi être utilisés pour obtenir un résultat analogue. On peut, par exemple, remplacer l'air par un gel.

D'autres combinaisons de solutions décrites précédemment sont possibles.

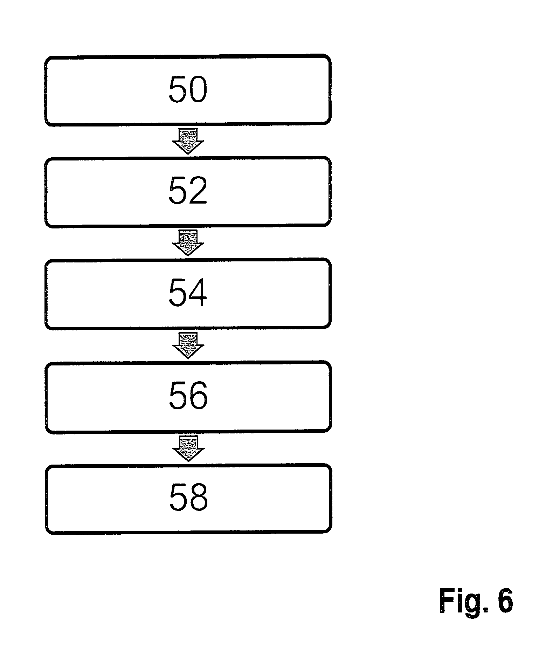

La coiffe 4 peut être réalisée selon le procédé de fabrication illustré schématiquement sur la figure 6.

Selon une étape 50, on découpe une couche de mousse 61, 62, 63, 64, 65, 106, 1065, 206, 2065 de polyuréthane et deux panneaux 41 et 42, ces derniers étant enduits, au moins sur les bords, d'un adhésif thermocollant.

Pour les panneaux 41 et 42, on utilise un film de polyuréthane, dont le panneau 41 est revêtu d'un velours pour permettre une fixation autoagrippante / détachable à l'intérieur du casque et dont le panneau 42 est revêtu d'un tissu du type polaire pour un bon confort de l'utilisateur.

Puis, selon une étape 52, on place l'empilement des deux panneaux 41 et 42 prenant en sandwich la couche de mousse 61, 62, 63, 64, 65, 106, 1065, 206, 2065 dans un moule de forme dont la profondeur est supérieure à l'épaisseur de la mousse 61, 62, 63, 64, 65, 106, 1065, 206, 2065.

Puis, on réalise selon une étape 54 une thermosoudure, opération de soudure de matériaux sous l'action de la chaleur. La thermosoudure est, par exemple, une soudure Haute Fréquence. On presse les deux panneaux 41 et 42, l'un contre l'autre, au niveau des bords périphériques. On applique ensuite une source de chaleur externe ou interne au matériau. On obtient alors une enveloppe étanche formant la poche 40.

Grâce au fait que le moule possède une profondeur plus importante que l'épaisseur de la mousse 61, 62, 63, 64, 65, 106, 1065, 206, 2065 cette dernière garde bien sa forme initiale (non comprimée).

Ensuite, lors d'une étape 56, on ménage une première ouverture dans la poche 40 ainsi formée et on fixe un élément 51, 1051, 2051 d'un moyen de réglage 5, 105, 205 du volume interne de la poche dans cette ouverture. Selon le positionnement de la poche 40 dans le casque et pour obtenir une meilleure accessibilité par l'utilisateur, l'élément du moyen de réglage 5, 105, 205 est une valve anti-retour 51, 1051, 2051 simple, soit il peut s'agir d'un ensemble formé par un conduit, par exemple un tuyau en matière plastique dans lequel est positionné une valve anti-retour 51, 1051, 2051.

En particulier lorsque le moyen de réglage 5, 105, 205 possède une commande d'inhibition 52, 1052, 2052 de la valve anti-retour combinée avec celle-ci, on préfère raccorder un tuyau à la poche avec sa valve anti-retour 51, 1051, 2051 pour faciliter l'accès à la commande.

Puis, selon une autre variante, on peut ménager selon une étape 58 une seconde ouverture dans la poche 40 et on soude ou colle un évent 52, 1052, 2052 commandable pour le regonflage de la poche 40. Ainsi, l'autogonflage et maintien en forme sont dissociés. Ce ceci peut être particulièrement intéressant lorsque la poche 2040 est couplée à une chambre de gonflage 208 comme montré sur la figure 5.

Alternativement, les étapes 56 et 58 peuvent être antérieures à l'étape 52 d'assemblage des panneaux. En effet, les éléments 51, 1051, 2051, 52, 1052, 2052 du moyen de réglage 5, 105, 205 peuvent être fixés sur un panneau 41, 42 en début de processus.

Le processus est décrit pour une poche 40 formée à partir de deux panneaux distincts 41, 42. Cette méthode s'applique mutatis mutandis à d'autres constructions de poche telles que l'utilisation d'un panneau replié dont les bords sont soudés les uns aux autres, ou l'utilisation d'un manchon soudé à ses extrémités.

On comprend donc que la poche 40 est un moyen de personnalisation et un moyen de confort qui se distingue par sa facilité d'utilisation et son caractère automatique. Ainsi, un utilisateur peut parfaitement adapter un casque de protection à sa morphologie propre.

## Revendications

1. Casque de protection (1, 101, 201) pour activité sportive comprenant
- au moins une couche d'amortissement (3) couvrant une partie du crâne (71) d'un utilisateur et,
- une coiffe (4, 104, 204) destinée à être interposée, au moins partiellement, entre ladite couche d'amortissement (3) et le crâne (71), la coiffe (4, 104, 204) comportant au moins une poche (40, 1040, 2040),
**caractérisé en ce que**
le volume interne de la poche définit, dans une configuration initiale de personnalisation, au moins localement, une épaisseur initiale de coiffe (Ei) et **en ce que** le casque (1) comprend un moyen de réglage (5, 105, 205) du volume interne de la poche permettant, suite à une compression de la poche, l'évacuation d'une partie du fluide contenu dans la poche, cette diminution du volume interne de la poche assurant une réduction localisée de l'épaisseur initiale de coiffe afin de conformer la coiffe à la morphologie du crâne.

2. Casque de protection selon la revendication 1, **caractérisé en ce que** le moyen de réglage (5, 105, 205) comprend un dispositif (51, 1051, 2051) permettant de maintenir une dépression du volume interne de la poche (40, 1040, 2040) par rapport à la pression du volume interne de la poche lorsqu'elle est dans une configuration initiale de personnalisation.

3. Casque de protection selon l'une des revendications précédentes, **caractérisé en ce que**, lorsque la poche (40, 1040, 2040) est dans sa configuration initiale, le volume interne de ladite poche est à pression ambiante.

4. Casque de protection selon l'une des revendications précédentes, **caractérisé en ce que** le moyen de réglage (5, 105, 205) comprend un dispositif (51, 1051, 2051) permettant l'évacuation automatique d'une partie du fluide contenu dans la poche (40, 1040, 2040) suite à une compression de ladite poche.

5. Casque de protection selon l'une des revendications précédentes, **caractérisé en ce que** le moyen de réglage (5, 105, 205) comprend une valve anti-retour (51, 1051, 2051) permettant uniquement de dégonfler la poche (40, 1040, 2040).

6. Casque de protection selon la revendication précédente, **caractérisé en ce que** le moyen de réglage (5, 105, 205) comprend un moyen d'inhibition (52, 1052, 2052) de la valve anti-retour (51, 1051, 2051) permettant une fonction évent pour le regonflage de la poche (40, 1040, 2040).

7. Casque de protection selon la revendication précédente, **caractérisé en ce que** seul le moyen d'inhibition (52, 1052, 2052) permet le regonflage de la poche (40, 1040, 2040) en connectant le volume interne de la poche à l'air ambiant

8. Casque de protection selon l'une des revendications précédentes, **caractérisé en ce que** la coiffe (4, 104, 204) comprend un moyen de regonflage (61, 62, 63, 64, 65, 106, 1065, 206, 2065) automatique de la poche (40, 1040, 2040) lorsque le volume interne de la poche (40, 1040, 2040) est connecté à l'air ambiant.

9. Casque de protection selon la revendication précédente, **caractérisé en ce que** le moyen de regonflage automatique est une mousse comprimable (61, 62, 63, 64, 65, 106, 1065, 206, 2065).

10. Casque de protection selon la revendication précédente, **caractérisé en ce que** la densité de la mousse comprimable (61, 62, 63, 64, 65, 106, 1065, 206, 2065) est comprise entre 20 kg/m3 et 50 kg/m3.

11. Casque de protection selon l'une des revendications 9 ou 10, **caractérisé en ce que** l'épaisseur de la mousse comprimable (61, 62, 63, 64, 65, 106, 1065, 206, 2065) est comprise entre 5 mm et 12 mm.

12. Casque de protection selon l'une des revendications précédentes, **caractérisé en ce que** la coiffe (104) couvre une bande sensiblement circonférentielle à l'intérieur du casque (101) correspondant à un tour de tête total ou partiel d'un utilisateur.

13. Casque de protection selon l'une des revendications précédentes, **caractérisé en ce que** la coiffe (104) comprend une surépaisseur dans la zone occipitale.

14. Casque de protection selon l'une quelconque des revendications précédentes, **caractérisé en ce qu'**il comporte une chambre de gonflage (208) disposée au niveau de la partie du casque destinée à être en contact avec la partie sous-occipitale du crâne de l'utilisateur, et **en ce qu'**un élément (2051) du moyen de réglage (205) de la poche (2040) est relié à cette chambre de gonflage (208) pour gonfler celle-ci, au moins partiellement, en cas de réduction volume du fluide contenu dans la poche (2040) lors de l'adaptation au moins partielle du casque (201) à la morphologie du crâne (71) de l'utilisateur.

15. Procédé de personnalisation d'un casque de protection (1), tel que défini dans les revendications précédentes, à la morphologie du crâne (71) d'un utilisateur comprenant les étapes suivantes :
- Initialiser la poche dans sa configuration initiale, dans cette configuration la circonférence interne de la coiffe est inférieure à la circonférence du crâne au niveau des zones de contact,
- Mise en place du casque sur la tête de l'utilisateur entrainant l'évacuation automatique, ou par une action manuelle, d'une partie du fluide contenu dans la poche suite à la compression de la poche afin de conformer la coiffe à la morphologie du crâne.
